(19) Europäisches Patentamt
European Patent Office
Office européen des brevets

(11) **EP 4 745 989 A1**

(12) **EUROPEAN PATENT APPLICATION**

(43) Date of publication:
**20.05.2026 Bulletin 2026/21**

(21) Application number: 25216872.9

(22) Date of filing: **19.11.2025**

(51) International Patent Classification (IPC):
**G16H 50/20** (2018.01)   **G16H 50/70** (2018.01)
**G06N 3/098** (2023.01)

(52) Cooperative Patent Classification (CPC):
**G16H 50/70; G06N 3/006; G06N 3/08; G06N 3/098;**
**G06N 3/126; G06N 5/01; G06N 7/01; G06N 20/00;**
**G16H 50/20;** G16H 10/60; G16H 15/00;
G16H 30/00; G16H 70/60

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH LA MA MD TN**

(30) Priority: **19.11.2024 CN 202411649562**

(71) Applicants:
• **Peking Union Medical College Hospital, Chinese**
  **Academy of Medical Sciences & Peking Union**
  **Medical College**
  **Beijing 100730 (CN)**
• **Digital Health China Technologies Co., Ltd.**
  **Beijing 100080 (CN)**

(72) Inventors:
• **ZHANG, Shuyang**
  **BEIJING, 100730 (CN)**
• **GUO, Jian**
  **BEIJING, 100730 (CN)**
• **JIN, Ye**
  **BEIJING, 100730 (CN)**
• **GONG, Mengchun**
  **BEIJING, 100080 (CN)**
• **SHI, Wenzhao**
  **BEIJING, 100080 (CN)**
• **ZHENG, Xihong**
  **BEIJING, 100080 (CN)**
• **LIU, Peng**
  **BEIJING, 100730 (CN)**

(74) Representative: **Comoglio, Elena**
  **Jacobacci & Partners S.p.A.**
  **Corso Emilia 8**
  **10152 Torino (IT)**

(54) **PROCESSING SYSTEM AND METHOD FOR RARE-DISEASE MEDICAL DATA**

(57)    A processing system and method for rare-disease medical data are provided, where a server, based on a medical data analysis request initiated by a target client, generates global model parameters and an initial analysis model, and sends the global model parameters and the initial analysis model to a plurality of clients; each client trains the initial analysis model based on client-side medical data of the client, and sends local model parameters and loss function values of the local analysis model to the server; the server inputs a plurality of the local model parameters and the loss function values received in a current round into a particle swarm optimization model, so as to obtain optimized model parameters output by the particle swarm optimization model; the server determines whether the optimized model parameters meet a preset condition, wherein if no, the optimized model parameters are sent to each client.

**EP 4 745 989 A1**

Description

## TECHNICAL FIELD

The present invention relates to the field of data processing technologies, and specifically to a processing system and a method for rare-disease medical data.

## BACKGROUND ART

With the development of science and technology, the analysis of medical data is increasingly reliant on AI technologies, such as analysis of disease types and disease conditions. However, due to the scarcity and confidentiality of rare-disease data, medical institutions often encounter a "data silo" problem when performing analysis of rare-disease data owing to their own insufficient data, which not only increases the difficulty of model training, but also imposes certain requirements on hardware resources of a diagnostic and treatment platform.

## SUMMARY

Embodiments of the present invention aim at providing a processing system and method for rare-disease medical data. Through integration of a federated learning model training mechanism and a particle swarm optimization algorithm, the processing system for the rare-disease medical data is constructed, which can ensure privacy of the rare-disease data while reducing training difficulty of a rare-disease data analysis model, and alleviate a data transmission load of the processing system.

In the first aspect, the present invention provides a processing system for rare-disease medical data, where the processing system includes a server and a plurality of clients, and each client is deployed at a different medical institution, wherein the server generates, based on a medical data analysis request initiated by a target client, global model parameters and an initial analysis model, and sends the global model parameters and the initial analysis model to the plurality of clients, wherein the medical data analysis request is configured to indicate a type of medical data and a rare-disease category to be analyzed by a medical institution corresponding to the target client, and the plurality of clients include the target client and other clients that have joined the medical data analysis request; each client trains the initial analysis model based on client-side medical data of the client, so as to obtain a local analysis model of the client, and sends local model parameters and loss function values of the local analysis model to the server, wherein the client-side medical data matches the type of medical data and the rare-disease category; the server inputs a plurality of the local model parameters and the loss function values received in a current round into a particle swarm optimization model, so as to obtain optimized model parameters output by the particle swarm optimization model; the server determines whether the optimized model parameters meet a preset condition, wherein if no, the optimized model parameters are sent to each client, so as to allow the plurality of clients to update the local analysis model, and if yes, the optimized model parameters are sent to the target client; and the target client inputs medical data to be tested, matching the type of medical data, into an optimized analysis model corresponding to the optimized analysis parameters, so as to acquire an analysis result of the rare-disease category output by the optimized analysis model.

In an embodiment, the server obtains the optimized model parameters output by the particle swarm optimization model in each round by a method as follows:

taking the local model parameter sent by each client in the current round as a position of particle i, and taking a loss function difference value of each client as a corresponding velocity of the particle i; determining whether a convergence condition is met; if the convergence condition is not met, updating the position and the velocity of each particle i, and returning to execute the step of determining whether the convergence condition is met; and if the convergence condition is met, outputting a personal best position and a global best position of each particle i as the optimized model parameters, wherein the loss function difference value is a difference value between the loss function value sent by the client in the current round and a mean of the loss function values in a previous round.

In an embodiment, the server sends the optimized model parameters in each round to each client by a method as follows:

determining a target particle j corresponding to the client among all particles output by the particle swarm optimization model, and sending a first optimized model parameter indicated by a personal best position of the target particle j and a second optimized model parameter indicated by the global best position to the client.

In an embodiment, the server sends the optimized model parameters in each round to each client by a method as follows:

determining third optimized model parameters indicated by personal best positions and fourth optimized model parameters indicated by global best positions of all particles i output by the particle swarm optimization model; sorting, for each model sub-parameter item among the optimized model parameters, sub-parameter absolute values corresponding to the model sub-parameter items among all the third optimized model parameters, so as to obtain top N1 sub-parameter values corresponding to the model sub-parameter; determining, for each model sub-parameter item among the optimized model parameters, a target direction and N2 sub-parameter values matching the target direction, based on a direction of top N1 sub-parameter values corresponding to the model sub-parameter item, a target direction and N2 sub-parameter values matching the target direction, where N2 is less than N1; calculating, for each model sub-parameter item among the optimized model parameters, a sub-parameter mean of N2 sub-parameter values corresponding to the model sub-parameter item; and performing fusion, for each model sub-parameter item among the optimized model parameters, on a sub-parameter mean of the model sub-parameter item and a sub-parameter value of the model sub-parameter item among the fourth optimized model parameters, to serve as a parameter value of the model sub-parameter item among the optimized model parameters, and sending the parameter value of the model sub-parameter item to the client.

In an embodiment, each client updates the model parameters of the local analysis model in each round by a method as follows: performing fusion based on received optimized model parameters and the client-side local model parameters, and calculating new local model parameters as updated model parameters of the local analysis model.

In an embodiment, the server generates an encryption key based on the optimized model parameters of the client in the previous round; and upon encryption of the optimized model parameters of the client in the current round through the encryption key, the encrypted optimized model parameters are sent to the client.

In an embodiment, the server generates the encryption key by a method as follows: multiplying parameter values of all sub-parameter items among the optimized model parameters, and calculating a comprehensive parameter value; and converting the comprehensive parameter value into a binary format, and padding to preset bits, so as to generate the encryption key.

In an embodiment, the server randomly generates, prior to the step of determining whether the convergence condition is met, a predetermined number of random model parameters and random loss function difference values, based on the local model parameters and loss function difference values sent by each client in the current round, as the position and the velocity of the particle i.

In an embodiment, the server determines whether the optimized model parameters meet a preset condition by a method as follows: determining whether a loss function value of the optimized analysis model corresponding to the optimized model parameters is less than a first preset threshold value, and if the loss function value of the optimized analysis model corresponding to the optimized model parameters is less than the first preset threshold value, determining that the optimized analysis model meets the preset condition; or determining whether number of rounds of iterations of the server is greater than a second preset threshold value, and if the number of rounds of iterations of the server is greater than the second preset threshold value, determining that the optimized model parameters meet the preset condition.

In the second aspect, the present invention provides a processing method for rare-disease medical data, including: generating, by a server, global model parameters and an initial analysis model based on a medical data analysis request initiated by a target client, and sending the global model parameters and the initial analysis model to a plurality of clients, wherein the medical data analysis request is configured to indicate a type of medical data and a rare-disease category to be analyzed by a medical institution corresponding to the target client, and the plurality of clients include the target client and other clients that have joined the medical data analysis request; training, by each client, the initial analysis model based on client-side medical data of the client, so as to obtain a local analysis model of the client, and sending local model parameters and loss function values of the local analysis model to the server, wherein the client-side medical data matches the type of medical data and the rare-disease category; inputting, by the server, a plurality of local model parameters and loss function values received in a current round into a particle swarm optimization model, so as to obtain optimized model parameters output by the particle swarm optimization model; determining, by the server, whether the optimized model parameters meet a preset condition, if the optimized model parameters do not meet the preset condition, sending the optimized model parameters to each client, so as to allow the plurality of clients to update the local analysis model, and if the optimized model parameters meet the preset condition, sending the optimized model parameters to the target client; and inputting, by the target client, medical data to be tested, matching the type of medical data, into an optimized analysis model corresponding to optimized analysis parameters, so as to acquire an analysis result of the rare-disease category output by the optimized analysis model.

The present invention provides a processing system and method for the rare-disease medical data. Through integration of a federated learning model training mechanism and a particle swarm optimization algorithm, a processing system for the rare-disease medical data is constructed, which can ensure privacy of the rare-disease data while reducing training difficulty of a rare-disease data analysis model, and alleviate a data transmission load of the processing system.

## BRIEF DESCRIPTION OF THE DRAWINGS

In order to more clearly illustrate technical solutions of embodiments of the present invention, drawings to be used in the embodiments of the present invention will be briefly introduced below. It is to be understood that the following drawings only show certain embodiments of the present invention, and thus should not be regarded as limitation to the scope. For those ordinarily skilled in the field, other relevant drawings also could be obtained according to these drawings without using inventive efforts.

FIG. 1 is a processing flowchart of a processing system for rare-disease medical data provided in embodiments of the present invention;
FIG. 2 is a flowchart of optimization steps for a particle swarm optimization model provided in embodiments of the present invention; and
FIG. 3 is a flowchart of steps for generating optimized model parameters through fusion of server provided in embodiments of the present invention.

**DETAILED DESCRIPTION OF EMBODIMENTS**

First, application scenarios of the present invention are described. Technical solutions of the present invention can be applied to a processing system for medical data, which specifically can be a processing system for medical data constructed based on federated learning. The processing system includes a server and a plurality of clients, with each client deployed at a different medical institution.

Existing federated learning model training mechanism is typically as follows: issuing, by a server, a model to clients of various medical institutions for client-side training, followed by aggregating models uploaded by various clients and performing model evaluation. Although this ensures the privacy and security of local data of the clients, the accuracy of the model is not high, and particularly in problem analysis scenarios of rare diseases with scarce sample data, the model training effect is relatively poor.

Technical solutions in embodiments of the present invention will be described below in conjunction with drawings in the embodiments of the present invention.

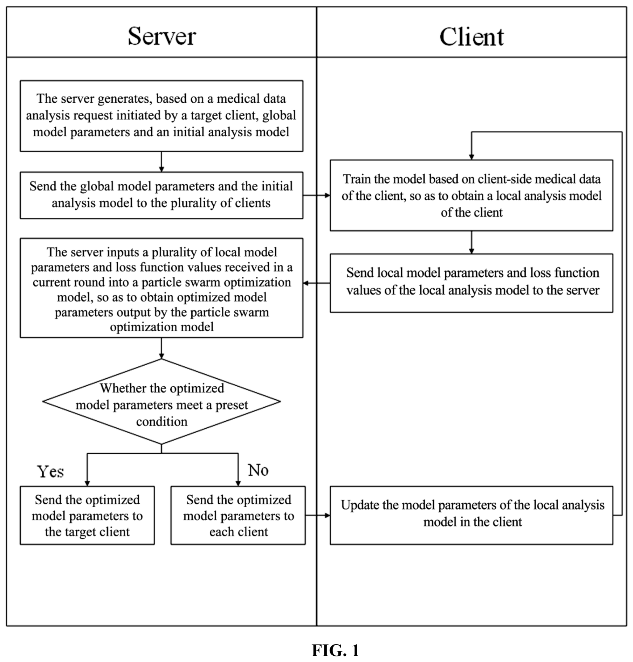

FIG. 1 is a processing flowchart of a processing system for rare-disease medical data provided in embodiments of the present invention.

The present invention provides a processing system for rare-disease medical data. The processing system implements model training based on a federated learning mechanism by a method as follows.

The server generates, based on a medical data analysis request initiated by a target client, global model parameters and an initial analysis model, and sends the global model parameters and the initial analysis model to the plurality of clients.

Herein, the medical data analysis request is configured to indicate a type of medical data and a rare-disease category to be analyzed by a medical institution corresponding to the target client, and the plurality of clients include the target client and other clients that have joined the medical data analysis request.

The target client herein is a target medical institution that initiates the medical data analysis request. After the medical data analysis request is sent to the server, the server broadcasts the medical data analysis request to the clients corresponding to the remaining medical institutions in the mechanism.

The remaining medical institutions can choose, at their own discretion, whether to respond to the medical data analysis request and assist the target medical institution in collaboratively training and generating an AI model with client-side medical data.

The types of medical data herein can include various types such as image pictures, case tables, record texts, and gene sequences. The rare-disease categories can be divided into a plurality of systems, such as hematological diseases, neurological diseases, cardiovascular diseases, urinary system diseases, endocrine system diseases, skeletal system diseases, respiratory system diseases, immune system diseases, digestive system diseases, ophthalmic system diseases, dermatological diseases, pediatric system diseases, and gynecological system diseases. Each system includes at least one disease. For example, the respiratory system diseases specifically include lymphangioleiomyomatosis, pulmonary alveolar proteinosis, and idiopathic pulmonary fibrosis.

Exemplarily, target medical institution A initiates a medical data analysis request, where the medical data analysis request indicates that a type of medical data to be analyzed is medical images, which specifically can be coronary CTA images, and the medical data analysis request indicates that a rare-disease category to be analyzed is coronary artery ectasia. That is, for an AI model to be constructed by the target medical institution A, input can be coronary CTA images of patients, and output can be analysis results such as probability value of having the disease, severity level of the disease, or identification of key areas affected by the disease.

The remaining medical institutions can choose, based on client-side medical data, whether to join the medical data analysis request of the target medical institution A.

Further, the server can determine the target medical institution A and other medical institutions B, C, D, and E having joined the medical data analysis request, and send the global model parameters and the initial analysis model to respective clients of the medical institutions A, B, C, D, and E.

The initial analysis model herein refers to an untrained basic neural network model, such as CNN (Convolutional Neural Networks) and BERT (Bidirectional Encoder Representations from Transformers) models.

The global model parameters herein are initial parameters, which specifically can include a convolution kernel size, a coefficient of a regularization term, number of layers of a neural network, a kernel function of a support vector machine, etc.

The server can choose an appropriate AI model and corresponding global model parameters based on the types of medical data and the rare-disease categories to be analyzed by the target medical institution, as indicated by the medical data analysis request. For example, when the type of medical data is medical images, CNN can be chosen as the initial analysis model, and when the type of medical data is time-series data such as electrocardiograms, RNN (Recurrent Neural Network) can be chosen as the initial analysis model. The global model parameters herein can be common initial values empirically set for models.

Each client trains the initial analysis model based on the client-side medical data of the client, so as to obtain a local analysis model of the client, and sends local model parameters and loss function values of the local analysis model to the server. The client-side medical data matches the type of medical data and the rare-disease category.

For respective clients of different medical institutions A, B, C, D, and E, the clients train the initial analysis model using the client-side medical data, and training modes, such as loss function of training and number of iterations, can be uniformly specified by the server, for example, 5 or 10 iterations per round, for a total of 10 rounds. For example, the medical institution A inputs coronary CTA images of patient a into the initial analysis model, trains the initial analysis model to output a probability value of patient a having the disease. Medical institution B can input coronary CTA images of patient b into the initial analysis model, and train the initial analysis model to output a probability value of the patient b having the disease. In the same manner, local analysis models and corresponding model parameters trained by respective clients with the client-side rare-disease data are obtained, respectively.

Exemplarily, the loss function can be selected from a mean squared error loss function, a cross-entropy loss function, and the like.

For example, in an embodiment of the present invention, the loss function can be represented by:

$$\mathcal{L}(\theta) = -\sum_{k=1}^{K} y'_k \cdot \hat{y}'_k + \lambda \|\theta\|_2^2.$$

Herein, $K$ represents the number of samples in the client-side medical data at the client, $y'_k$ represents a correct result of a $k^{th}$ sample, $\hat{y}'_k$ represents a predicted result of the $k^{th}$ sample, $\lambda$ is a regularization coefficient, and $\theta$ represents a model parameter.

Upon completion of each round of iteration, each client can feed back the local model parameters and the loss function value of a current local analysis model to the server, which specifically can be represented by

$$S_A = \{s_A^1, \ s_A^2, \ s_A^3, \ s_A^4, \ Lost_A\}, S_B = \{s_B^1, \ s_B^2,$$

$$s_B^3, \ s_B^4, \ Lost_B\}, ..., S_E = \{s_E^1, \ s_E^2, \ s_E^3, \ s_E^4, \ Lost_E\},$$ where $S^1, S^2, S^3,$ and $S^4$ are a plurality of different model sub-parameter items of local analysis models, and $Lost$ is the loss function value.

The server inputs all local model parameters and loss function values received in the current round into a particle swarm optimization model, so as to obtain optimized model parameters output by the particle swarm optimization model.

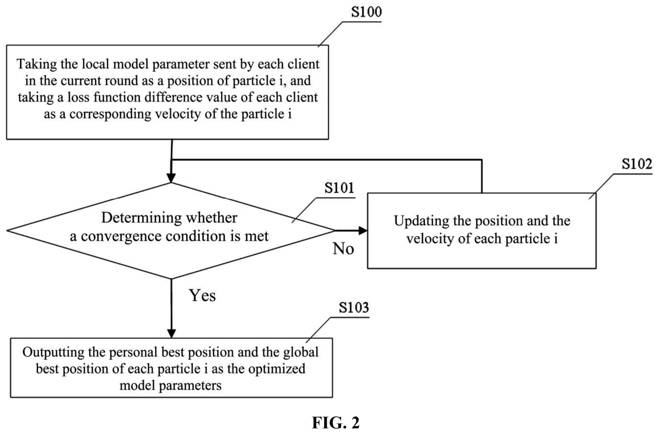

For example, $S_A$-$S_E$ received by the server in each round are input into the particle swarm optimization model, and as shown in FIG. 2, the particle swarm optimization model outputs the optimized model parameters in each round by a method as follows.

S100, taking the local model parameter sent by each client in the current round as a position of particle i, and taking a loss function difference value of each client as a corresponding velocity of the particle i.

Herein, the loss function difference value is a difference value between the loss function value sent by the client in the current round and a mean of the loss function values in a previous round.

For each particle i, the position thereof can be represented by

$$X_A = \{s_A^1, \ s_A^2, \ s_A^3, \ s_A^4\}, X_B = \{s_B^1, \ s_B^2, \ s_B^3, \ s_B^4\}, ..., X_E = \{s_E^1, \ s_E^2, \ s_E^3, \ s_E^4\}.$$

For each particle i, the velocity thereof can be represented by $V_A = \overline{Lost}' - Lost_A$, $V_B = \overline{Lost}' - Lost_B$, ..., $V_E = \overline{Lost}' - Lost_E$, wherein $\overline{Lost}'$ is a mean of the loss function values corresponding to all clients in the previous round.

S101, determining whether a convergence condition is met.

The convergence condition herein can be the number of iterations, etc.

Moreover, a fitness value of each particle is calculated through an objective function, and compared with a personal (particle) best position, so as to determine the personal best position of the particle, and a global best position. The objective function herein can be determined through a loss function of a pre-training model.

For example, the objective function can be expressed as:

$$\begin{cases} \min \mathcal{L}(\theta) = -\sum_{k=1}^{K} y_k' \cdot \hat{y}_k' + \lambda \|\theta\|_2^2 \\ s.t. \ \theta \in \Omega \\ t1 \leq \mathcal{L}(\theta) \leq t2 \end{cases}$$

Herein, $\Omega$ is a value range of the model parameter $\theta$ (which also can be a particle position), and t1 and t2 are value ranges of the loss function.

S102, if no, updating the position and the velocity of each particle i, and returning to execute the step of determining whether the convergence condition is met.

In step S102, the position and the velocity of each particle i can be updated by a method as follows:

$$V_i' = V_i + C_1 r_1 \ (pbest_i - X_i) \ + C_2 r_2 \ (gbest_i - X_i) \ ;$$

$$X_i' = X_i + V_i.$$

Herein, $V_i'$ is velocity of the particle i after updating, $V_i$ is velocity of the particle i before updating, $X_i'$ is position of the particle i after updating, $X_i$ is position of the particle i before updating, $pbest_i$ is personal best position of the particle i, $gbest_i$ is global best position, $C_1$ and $C_2$ are learning parameter values (generally $C_1 = C_2 = 2$), and $r_1$ and $r_2$ are random parameter values, with a value of [0, 1], where i = 1, 2, 3... N, N being the total number of particles.

S103, if yes, outputting the personal best position and the global best position of each particle i as the optimized model parameters.

Finally, when the convergence condition is met, the personal best position and the global best position for each particle i can be obtained.

The server determines whether the optimized model parameters meet a preset condition, wherein if no, the optimized model parameters are sent to each client, so as to allow the plurality of clients to update the local analysis model, and if yes, the optimized model parameters are sent to the target client.

The server can take the optimized model parameters as model parameters of the initial analysis model, so as to determine an optimized analysis model, and then can calculate accuracy of the optimized analysis model. When the accuracy meets requirements, iteration can be terminated, and the obtained optimized analysis model is sent to the target client. When the accuracy is not met, the optimized model parameters are fed back to each client, enabling the clients to continue iterative training.

The server can further determine whether the optimized model parameters meet a preset condition by a method as follows:

determining whether a loss function value of the optimized analysis model corresponding to the optimized model parameters is less than a first preset threshold value, and if yes, determining that the optimized analysis model meets the preset condition; or determining whether the number of rounds of iterations of the server is greater than a second preset threshold value, and if yes, determining that the optimized model parameters meet the preset condition. For example, when the server has executed step 3 for 80 times, it can be determined that the optimized analysis model meets the preset condition.

The target client inputs medical data to be tested, matching the type of medical data, into the optimized analysis model corresponding to the optimized analysis parameters, so as to acquire an analysis result corresponding to the rare-disease category output by the optimized analysis model.

The target client can obtain a corresponding analysis result through a final optimized analysis model. The optimized analysis model herein has higher accuracy and better generalization ability, and further provides a better assisting effect on the analysis of rare diseases.

In an embodiment of the present invention, the server can send the optimized model parameters in each round to each client by a method as follows:

determining a target particle j corresponding to the client among all particles output by the particle swarm optimization model, and sending a first optimized model parameter indicated by a personal best position of the target particle j and a second optimized model parameter indicated by a global best position to the client.

In this embodiment, the server determines the first optimized model parameter indicated by the personal best position and the second optimized model parameter indicated by the global best position of each particle and sends the first optimized model parameter and the second optimized model parameter to the client corresponding to the particle.

Exemplarily, the position and the velocity of the target particle j adopt $S_A = \{s_A^1, s_A^2, s_A^3, s_A^4, Lost_A\}$, and then the first optimized model parameter and the second optimized model parameter corresponding to the target particle j are sent to the client of the medical institution A.

Each client can update the model parameters of the local analysis model in each round by a method as follows:

performing fusion based on the received optimized model parameters and a client-side local model parameters, and calculating new local model parameters as updated model parameters of the local analysis model.

Exemplarily, the medical institution A can perform weighted fusion (may also perform averaging, etc.) on the first optimized model parameter and the second optimized model parameter, so as to obtain fused local model parameters as updated model parameters of the initial analysis model. A weight value herein can be specified in advance.

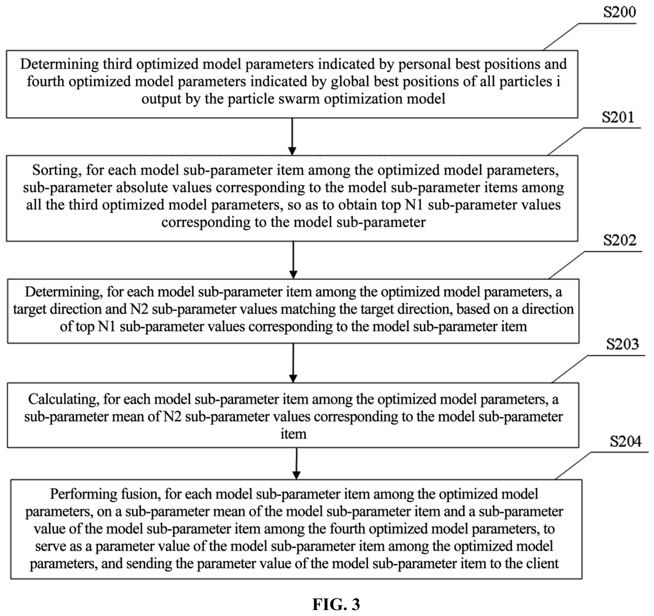

In an embodiment of the present invention, as shown in FIG. 3, the server can also send the optimized model parameters in each round to each client by a method as follows:

S200, determining third optimized model parameters indicated by personal best positions and fourth optimized model parameters indicated by global best positions of all particles i output by the particle swarm optimization model.

The third optimized model parameters may include $X_A^{pbest} = \{s_A^1, s_A^2, s_A^3, s_A^4\}$, $X_B^{pbest} = \{s_B^1, s_B^2, s_B^3, s_B^4\}, \ldots$, and $X_E^{pbest} = \{s_E^1, s_E^2, s_E^3, s_E^4\}$.

S201, sorting, for each model sub-parameter item among the optimized model parameters, sub-parameter absolute values corresponding to the model sub-parameter items among all the third optimized model parameters, so as to obtain top N1 sub-parameter values corresponding to the model sub-parameter.

Exemplarily, for a first model sub-parameter item among the optimized model parameters, absolute values of $s_A^1, s_B^1, s_C^1, s_D^1$, and $s_E^1$ are sorted, and $|s_A^1| = 5.2, |s_D^1| = 5.0, |s_C^1| = 4.95$, $|s_B^1| = 4.8$, and $|s_E^1| = 2.45$ can be obtained.

For a second model sub-parameter item among the optimized model parameters, absolute values of $s_A^2, s_B^2, s_C^2, s_D^2$, and $s_E^2$ can be sorted, and $|s_E^2|, |s_C^2|, |s_B^2|, |s_A^2|$, and $|s_D^2|$ can be obtained. The same process is applied to the remaining.

Next, sorted sequences are pruned, that is, only top K% parameter values are kept, and the pruned parameter values are zeroed out.

For example, $|s_A^1| = 5.2, |s_D^1| = 5.0, |s_C^1| = 4.95, |s_B^1| = 4.8, |s_E^1| = 0$, and N1 = 4.

S202, for each model sub-parameter item among the optimized model parameters, determining, based on a direction of top N1 sub-parameter values corresponding to the model sub-parameter item, a target direction and N2 sub-parameter values matching the target direction, where N2 is less than N1.

For the parameter value of each parameter item, differentiation can be performed based on a sign of the parameter value (+1 or -1).

Exemplarily, for the first model sub-parameter item among the optimized model parameters, $s_A^1 = +5.2, s_B^1 = -5, s_C^1 = +4.95$, and $s_D^1 = -4.8$, the target direction herein is a sign direction with the highest total magnitude. For example, the magnitude in a positive direction is 5.2 + 4.95 and the magnitude in a negative direction is 4 + 4.8, and then the target direction is the positive direction.

Further, $s_A^1 = +5.2$ and $s_C^1 = +4.95$ are determined, where N2 = 2.

Other model sub-parameter terms also match respectively.

S203, calculating, for each model sub-parameter item among the optimized model parameters, a sub-parameter mean of N2 sub-parameter values corresponding to the model sub-parameter item.

Exemplarily, for the first model sub-parameter item among the optimized model parameters, a sub-parameter mean between $s_A^1 = +5.2$ and $s_C^1 = +4.95$ is calculated as $|S^1| = 5.075$.

S204, for each model sub-parameter item among the optimized model parameters, performing fusion on a sub-parameter mean of the model sub-parameter item and a sub-parameter value of the model sub-parameter item among the fourth optimized model parameters, to serve as a parameter value of the model sub-parameter item among the optimized model parameters, and sending the parameter value of the model sub-parameter item to the client.

In step S204, taking the first model sub-parameter item among the optimized model parameters as an example, the fusion can be performed on the sub-parameter mean and the sub-parameter value of the model sub-parameter item among the fourth optimized model parameters by a method as follows:

$$S_{mul}^1 = |S^1| + \mu S_{whole}^1.$$

Herein, $S_{mul}^1$ is a sub-parameter value of the first model sub-parameter item among the optimized model parameters, $|S^1|$ is a sub-parameter mean of the first model sub-parameter item obtained in step S203, $S_{whole}^1$ is a sub-parameter value of a first model sub-parameter item among the fourth optimized model parameters, and $\mu$ is a scaling coefficient, and can be set as needed.

In this way, the optimized model parameters received by the clients can be represented as $\{S_{mul}^1 \text{、} S_{mul}^2 \text{、} S_{mul}^3 \text{、} S_{mul}^4\}$, the clients can directly update them as the model parameters of the local analysis models, without processing the received optimized model parameters prior to use, which can reduce the occupation of local resources at the clients, minimize communication consumption between the server and the clients, and can be downward compatible with some medical institutions with relatively low configurations of client operating environment, thereby broadening the potential for the medical institutions to participate in the training, and increasing the number of training samples.

Due to the low incidence of rare diseases, fewer samples can be obtained. In order to improve accuracy of a model training process, in an embodiment of the present invention, in step S100, in addition to using real clients as particles, the number of particles can also be expanded. That is, prior to the step of determining whether the convergence condition is met, the server further performs a step of randomly generating a predetermined number of random model parameters and random loss function difference values, based on the local model parameters and loss function difference values sent by each client in a current round, as the position and velocity of the particle i.

Herein, based on the local model parameters and the loss function difference values iterated by real clients, such as, $\{s_A^1 = +5.2, s_A^2 = -0.2, s_A^3 = +3.2, s_A^4 = +1.2, V_A = +0.8\}$, increasing and decreasing within a specified range (such as 0.5 and 0.2) can be performed, and {+5.0, -0.4, +3.0, 1.4, 0.6}, etc. correspondingly obtained are taken as the position and the velocity of the particle i.

In this way, a search scope of a particle swarm optimization algorithm is expanded, thus enabling the clients to obtain a more accurate local analysis model in each round.

Since the data of rare diseases involve patient privacy, in order to further protect privacy and security of local data at each client, in an embodiment of the present invention, the server can generate an encryption key based on the optimized model parameters of the client in the previous round. The server encrypts the optimized model parameters of the client in the current round through an encryption key, and sends the encrypted optimized model parameters to the client. In this way, the client can decrypt the optimized model parameters in the current round sent by the server through the locally stored optimized model parameters in the previous round. In this way, theft of the model parameters can be prevented, ensuring the security of the medical data of patients.

Specifically, the server can generate the encryption key by a method as follows:

multiplying parameter values of all sub-parameter items among the optimized model parameters, and calculating a comprehensive parameter value; and converting the comprehensive parameter value into a binary format, and padding to preset bits, so as to generate the encryption key.

Exemplarily, based on the optimized model parameters $\{S_{mul}^1, S_{mul}^2, S_{mul}^3, S_{mul}^4\}$ sent in the previous round, the server calculates $S_{mul}^1 \times S_{mul}^2 \times S_{mul}^3 \times S_{mul}^4$ as the comprehensive parameter value, and then converts the comprehensive parameter value into binary. The binary is padded, for example, to 128 bits by adding 0. Further, the server can adopt the AES encryption algorithm to encrypt the optimized model parameters to be sent in the current round, and send the encrypted optimized model parameters to a corresponding client.

An embodiment of the present invention further provides a processing method for rare-disease medical data, applicable to the processing system for the rare-disease medical data, including:

generating, by a server, global model parameters and an initial analysis model based on a medical data analysis request initiated by a target client, and sending the global model parameters and the initial analysis model to a plurality of clients, wherein the medical data analysis request is configured to indicate a type of medical data and a rare-disease category to be analyzed by a medical institution corresponding to the target client, and the plurality of clients include the target client and other clients that have joined the medical data analysis request;

training, by each client, the initial analysis model based on the client-side medical data of the client, so as to obtain a local analysis model of the client, and sending local model parameters and loss function values of the local analysis model to the server, wherein the client-side medical data matches the type of medical data and the rare-disease category;

inputting, by the server, a plurality of local model parameters and loss function values received in a current round into a particle swarm optimization model, so as to obtain optimized model parameters output by the particle swarm optimization model;

determining, by the server, whether the optimized model parameters meet a preset condition, if no, sending the optimized model parameters to each client, so as to allow the plurality of clients to update the local analysis model, and if yes, sending the optimized model parameters to the target client; and

inputting, by the target client, medical data to be tested, matching the type of medical data, into the optimized analysis model, so as to acquire an analysis result of the rare-disease category output by the optimized analysis model.

The present invention provides a processing method for rare-disease medical data. Through integration of a federated learning model training mechanism and a particle swarm optimization algorithm, a processing system for the rare-disease medical data is constructed, which can ensure privacy of the rare-disease data while reducing training difficulty of a rare-disease data analysis model, and alleviate a data transmission load of the processing system.

In the embodiments provided in the present invention, it should be understood that the disclosed apparatus and method may be implemented in other manners. The apparatus embodiments described in the above are merely exemplary. For example, units are merely divided according to logical functions, but they may be divided in other ways in practical implementation. For another example, a plurality of units or components can be combined or integrated into another system, or some features can be omitted, or not executed. In addition, a mutual coupling, direct coupling or communication connection shown or discussed can be an indirect coupling or communication connection through some communication interfaces, devices or units, and can be in an electrical form, a mechanical form or other forms.

In addition, the units described as separate components may be or may not be physically separated. The components shown as units may be or may not be physical units, i.e., they may be located at one place or distributed onto multiple network units. Some or all of the units can be selected as actually needed to achieve the objectives of the solutions in the embodiments.

Furthermore, individual functional modules in each embodiment of the present invention can be integrated together to form an independent part, or each module may exist alone, or two or more modules may be integrated to form an independent part.

It should be noted that if the function is realized in a form of software functional module and is sold or used as an individual product, it may be stored in one computer-readable storage medium. Based on such understanding, the technical solutions of the present invention in essence or parts making contribution to the prior art or parts of the technical solutions can be embodied in form of a software product, and this computer software product is stored in a storage medium, including several instructions for making a computer device (which may be a personal computer, a server or a network device, etc.) execute all or part of the steps of method of the embodiments of the present invention. The aforementioned storage medium includes various media that can store program codes, such as USB flash disk, mobile hard disk, read-only memory (ROM), random access memory (RAM), diskette and compact disk.

Herein, the relational terms such as first and second are used only to distinguish one entity or operation from another entity or operation, rather than necessarily requiring or implying any such actual relationship or sequence between these entities or operations.

The above-mentioned are merely for the embodiments of the present invention and not intended to limit the scope of protection of the present invention. For those skilled in the art, various modifications and changes could be made to the present invention. Any modifications, equivalent substitutions, improvements and so on made within the spirit and principle of the present invention should be covered within the scope of protection of the present invention.

**Claims**

1. A processing system for rare-disease medical data, **characterized by** comprising a server and a plurality of clients, each client being deployed at a different medical institution, wherein

    the server generates, based on a medical data analysis request initiated by a target client, global model parameters and an initial analysis model, and sends the global model parameters and the initial analysis model to the plurality of clients, wherein the medical data analysis request is configured to indicate a type of medical data and a rare-disease category to be analyzed by a medical institution corresponding to the target client, and the plurality of clients comprise the target client and other clients that have joined the medical data analysis request;

    each client trains the initial analysis model based on client-side medical data of the client, so as to obtain a local analysis model of the client, and sends local model parameters and loss function values of the local analysis model to the server, wherein the client-side medical data matches the type of medical data and the rare-disease category;

    the server inputs a plurality of the local model parameters and the loss function values received in a current round into a particle swarm optimization model, so as to obtain optimized model parameters output by the particle swarm optimization model;

    the server determines whether the optimized model parameters meet a preset condition, wherein if the optimized model parameters do meet the preset condition, the optimized model parameters are sent to each client so as to allow the plurality of clients to update the local analysis model, and if the optimized model parameters meet the preset condition, the optimized model parameters are sent to the target client; and

    the target client inputs medical data to be tested, matching the type of medical data, into an optimized analysis model corresponding to the optimized model parameters, so as to acquire an analysis result of the rare-disease category output by the optimized analysis model.

2. The system according to claim 1, wherein the server obtains the optimized model parameters output by the particle swarm optimization model in each round by a method as follows:

    taking the local model parameter sent by each client in the current round as a position of particle i, and taking a loss function difference value of each client as a corresponding velocity of the particle i;

    determining whether a convergence condition is met;

    if the convergence condition is not met, updating the position and the velocity of each particle i, and returning to execute the step of determining whether the convergence condition is met; and

    if the convergence condition is met, outputting a personal best position and a global best position of each particle i as the optimized model parameters, wherein

    the loss function difference value is a difference value between the loss function value sent by the client in the current round and a mean of the loss function values in a previous round.

3. The system according to claim 2, wherein the server sends the optimized model parameters in each round to each client by a method as follows:
    determining a target particle j corresponding to the client among all particles output by the particle swarm optimization model, and sending a first optimized model parameter indicated by a personal best position of the target particle j and a second optimized model parameter indicated by the global best position to the client.

4. The system according to claim 2, wherein the server sends the optimized model parameters in each round to each client by a method as follows:

    determining third optimized model parameters indicated by personal best positions and fourth optimized model parameters indicated by global best positions of all particles i output by the particle swarm optimization model;

    sorting, for each model sub-parameter item among the optimized model parameters, sub-parameter absolute values corresponding to the model sub-parameter items among all the third optimized model parameters, so as to obtain top N1 sub-parameter values corresponding to the model sub-parameter;

    determining, for each model sub-parameter item among the optimized model parameters, a target direction and N2 sub-parameter values matching the target direction, based on a direction of top N1 sub-parameter values corresponding to the model sub-parameter item, wherein N2 is less than N1;

    calculating, for each model sub-parameter item among the optimized model parameters, a sub-parameter mean of N2 sub-parameter values corresponding to the model sub-parameter item; and

    performing fusion, for each model sub-parameter item among the optimized model parameters, on a sub-parameter mean of the model sub-parameter item and a sub-parameter value of the model sub-parameter item among the fourth optimized model parameters, to serve as a parameter value of the model sub-parameter item among the optimized model parameters, and sending the parameter value of the model sub-parameter item to the client.

5. The system according to claim 3 or 4, wherein each client updates the model parameters of the local analysis model in each round by a method as follows:
performing fusion based on received optimized model parameters and client-side local model parameters, and calculating new local model parameters as updated model parameters of the local analysis model.

6. The system according to claim 3 or 4, wherein the server generates an encryption key based on the optimized model parameters of the client in the previous round; and
upon encryption of the optimized model parameters of the client in the current round through the encryption key, the encrypted optimized model parameters are sent to the client.

7. The system according to claim 6, wherein the server generates the encryption key by a method as follows:

   multiplying parameter values of all sub-parameter items among the optimized model parameters, and calculating a comprehensive parameter value; and

   converting the comprehensive parameter value into a binary format, and padding to preset bits, so as to generate the encryption key.

8. The system according to claim 2, wherein the server randomly generates, prior to the step of determining whether the convergence condition is met, a predetermined number of random model parameters and random loss function difference values, based on the local model parameters and loss function difference values sent by each client in the current round, as the position and the velocity of the particle i.

9. The system according to claim 2, wherein the server determines whether the optimized model parameters meet a preset condition by a method as follows:

   determining whether a loss function value of the optimized analysis model corresponding to the optimized model parameters is less than a first preset threshold value, and if the loss function value of the optimized analysis model corresponding to the optimized model parameters is less than the first preset threshold value, determining that the optimized analysis model meets the preset condition; or

   determining whether number of rounds of iterations of the server is greater than a second preset threshold value, and if the number of rounds of iterations of the server is greater than the second preset threshold value, determining that the optimized model parameters meet the preset condition.

10. A processing method for rare-disease medical data, **characterized by** comprising:

   generating, by a server, global model parameters and an initial analysis model based on a medical data analysis request initiated by a target client, and sending the global model parameters and the initial analysis model to a plurality of clients, wherein the medical data analysis request is configured to indicate a type of medical data and a rare-disease category to be analyzed by a medical institution corresponding to the target client, and the plurality of clients include the target client and other clients that have joined the medical data analysis request;
   training, by each client, the initial analysis model based on client-side medical data of the client, so as to obtain a local analysis model of the client, and sending local model parameters and loss function values of the local analysis model to the server, wherein the client-side medical data matches the type of medical data and the rare-disease category;
   inputting, by the server, a plurality of local model parameters and loss function values received in a current round into a particle swarm optimization model, so as to obtain optimized model parameters output by the particle swarm optimization model;
   determining, by the server, whether the optimized model parameters meet a preset condition, if the optimized model parameters do not meet the preset condition, sending the optimized model parameters to each client, so as to allow the plurality of clients to update the local analysis model, and if the optimized model parameters meet the preset condition, sending the optimized model parameters to the target client; and
   inputting, by the target client, medical data to be tested, matching the type of medical data, into an optimized analysis model corresponding to optimized analysis parameters, so as to acquire an analysis result of the rare-disease category output by the optimized analysis model.

| Server | Client |
|---|---|
| The server generates, based on a medical data analysis request initiated by a target client, global model parameters and an initial analysis model | |
| Send the global model parameters and the initial analysis model to the plurality of clients | Train the model based on client-side medical data of the client, so as to obtain a local analysis model of the client |
| The server inputs a plurality of local model parameters and loss function values received in a current round into a particle swarm optimization model, so as to obtain optimized model parameters output by the particle swarm optimization model | Send local model parameters and loss function values of the local analysis model to the server |
| Whether the optimized model parameters meet a preset condition | |
| Yes — Send the optimized model parameters to the target client   No — Send the optimized model parameters to each client | Update the model parameters of the local analysis model in the client |

**FIG. 1**

S100

Taking the local model parameter sent by each client in the current round as a position of particle i, and taking a loss function difference value of each client as a corresponding velocity of the particle i

S101

Determining whether a convergence condition is met

S102

Updating the position and the velocity of each particle i

No

Yes

S103

Outputting the personal best position and the global best position of each particle i as the optimized model parameters

**FIG. 2**

S200

Determining third optimized model parameters indicated by personal best positions and fourth optimized model parameters indicated by global best positions of all particles i output by the particle swarm optimization model

S201

Sorting, for each model sub-parameter item among the optimized model parameters, sub-parameter absolute values corresponding to the model sub-parameter items among all the third optimized model parameters, so as to obtain top N1 sub-parameter values corresponding to the model sub-parameter

S202

Determining, for each model sub-parameter item among the optimized model parameters, a target direction and N2 sub-parameter values matching the target direction, based on a direction of top N1 sub-parameter values corresponding to the model sub-parameter item

S203

Calculating, for each model sub-parameter item among the optimized model parameters, a sub-parameter mean of N2 sub-parameter values corresponding to the model sub-parameter item

S204

Performing fusion, for each model sub-parameter item among the optimized model parameters, on a sub-parameter mean of the model sub-parameter item and a sub-parameter value of the model sub-parameter item among the fourth optimized model parameters, to serve as a parameter value of the model sub-parameter item among the optimized model parameters, and sending the parameter value of the model sub-parameter item to the client

**FIG. 3**

## EUROPEAN SEARCH REPORT

**Application Number**

EP 25 21 6872

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 116 741 388 A (CHINESE PLA GENERAL HOSPITAL) 12 September 2023 (2023-09-12) * the whole document * | 1-10 | INV. G16H50/20 G16H50/70 G06N3/098 |
| A | CN 114 566 277 A (CHINA PETROLEUM UNIV EAST CHINA) 31 May 2022 (2022-05-31) * the whole document * | 1-10 | |
| A | CN 115 408 377 A (BEIJING ZHIYUAN ARTIFICIAL INTELLIGENCE RES INSTITUTE) 29 November 2022 (2022-11-29) * the whole document * | 1-10 | |
| A | SIGANPORIA AFREEN ET AL: "Privacy-Enhanced Federated Learning for Rare Genetic Disorder Classification with EHR", 2023 GLOBAL CONFERENCE ON INFORMATION TECHNOLOGIES AND COMMUNICATIONS (GCITC) IEEE, 1 December 2023 (2023-12-01), pages 1-8, XP034590233, DOI: 10.1109/GCITC60406.2023.10426518 ISBN: 9798350308143 [retrieved on 2024-04-18] * abstract * | 1-10 | **TECHNICAL FIELDS SEARCHED (IPC)** G06E G06F G06N G16B G16H |

The present search report has been drawn up for all claims

| Place of search | Date of completion of the search | Examiner |
|---|---|---|
| Munich | 27 March 2026 | Lorenz, Larissa |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 21 6872

This annex lists the patent family members relating to the patent documents cited in the above-mentioned European search report.
The members are as contained in the European Patent Office EDP file on
The European Patent Office is in no way liable for these particulars which are merely given for the purpose of information.

27-03-2026

| Patent document cited in search report | | Publication date | Patent family member(s) | Publication date |
|---|---|---|---|---|
| CN 116741388 | A | 12-09-2023 | NONE | |
| CN 114566277 | A | 31-05-2022 | NONE | |
| CN 115408377 | A | 29-11-2022 | NONE | |

EPO FORM P0459

For more details about this annex : see Official Journal of the European Patent Office, No. 12/82